# DEMANDE DE BREVET EUROPEEN

(11) **EP 2 881 470 A1**
(43) Date de publication de la demande: **10.06.2015**
(21) Numéro de dépôt: 13306674.6
(22) Date de dépôt: 05.12.2013
(51) Int. Cl.: C12Q 1/26, C12N 9/02

(54) **Microsomes irreversiblement inhibés pour des CYP450 leurs utilisations dans le phénotypage enzymatique des voies métaboliques**

(71) Demandeur: Les Laboratoires Servier, 92284 Suresnes Cedex (FR)
(72) Inventeur: Carardec, Fabrice, 45450 Donnerie (FR); Parmentier, Yannick, 45560 Saint-Denis-en-Val (FR); Pothier, Corinne, 45800 Saint-Jean-De-Braye (FR)

(57) **Abrégé**

Méthode de préparation de microsomes isolés comprenant un cytochrome P450 (CYP450) irréversiblement inhibé. Microsome isolé caractérisé dont un cytochrome P450 est irréversiblement inhibé par un inhibiteur non réversible. Utilisation des microsomes isolés selon l'invention dans une méthode de phénotypage des réactions enzymatiques d'un candidat médicament.

## Description

L'invention s'inscrit dans le cadre de la recherche et de la mise au point de produits et méthodes pour l'évaluation des interactions médicamenteuses des nouveaux médicaments. La présente invention concerne une méthode de préparation de microsomes isolés irréversiblement inhibés pour un cytochrome P450 humain spécifique (CYP450) qui seront utilisés pour quantifier la contribution de cette enzyme dans le métabolisme de principes actifs.

L'efficacité et la toxicité d'un médicament peuvent être modifiées par l'administration d'un autre composé : médicament, polluant environnemental, aliment. Il s'agit d'interactions médicamenteuses (DDI : Drug Drug Interactions). Il existe différents types de mécanismes d'interactions, la plus importante étant l'interaction métabolique appartenant au groupe des interactions pharmacocinétiques.
Par interaction médicamenteuse métabolique on entend entre autre le fait qu'un médicament A peut modifier le métabolisme d'un médicament B co-administré soit en accélérant le métabolisme de B (activation ou induction) soit en le réduisant (inhibition ou répression). De cette interaction médicamenteuse métabolique va découler dans le programme de développement d'un nouveau médicament d'une part, l'identification des enzymes impliquées dans le métabolisme du principe actif et d'autre part, le potentiel inhibiteur ou inducteur dudit principe actif.
Le foie est le principal site du métabolisme des médicaments. L'hépatocyte contient des enzymes essentielles du métabolisme parmi lesquelles les cytochromes P450 (CYP450). Les cytochromes P450 constituent donc la cible majeure dans la prédiction des interactions médicamenteuses.
Afin de prédire le risque d'interactions médicamenteuses, il faut identifier et déterminer la contribution de chaque enzyme dans le métabolisme du principe actif : c'est le phénotypage des réactions enzymatiques.
Pour estimer la contribution de chaque enzyme dans le métabolisme du principe actif différentes méthodes de phénotypages des voies métaboliques peuvent être mises en oeuvre.

L'utilisation d'une banque de microsomes de foies humains caractérisés peut aider à l'identification des enzymes impliquées dans le métabolisme d'un principe actif. Cette méthode nécessite une caractérisation préalable de l'activité enzymatique des principaux CYP450 d'au moins quinze lots individuels de microsomes issus de foies humains. Chacun des quinze lots de microsomes est incubé avec le principe actif afin de déterminer une corrélation entre sa vitesse de métabolisme et l'activité de chaque cytochrome P450 de ces mêmes microsomes. Cependant l'utilisation des banques microsomales ne permet pas une détermination quantitative des enzymes impliquées, la corrélation étant difficile à réaliser.

Les enzymes recombinantes sont également utilisées pour estimer la contribution relative de chaque cytochrome P450 dans le métabolisme du principe actif. Le niveau d'expression du cytochrome P450 dans le système recombinant étant différent, souvent beaucoup plus élevé, que dans les microsomes de foie humain natif, il est nécessaire d'intégrer un facteur correctif pour extrapoler la contribution de chaque CYP450 dans les microsomes recombinants par rapport aux microsomes humains (facteur de correction = Relative Activity Factor). Ce facteur de correction doit être calculé par mesure expérimentale de l'activité enzymatique de chaque CYP450 testé d'une part, sur les microsomes humains natifs et d'autre part, sur les microsomes recombinants. Cette approche permet d'évaluer indirectement de manière sélective et semi-quantitative la contribution relative de chaque enzyme dans le métabolisme du principe actif.

Cependant de par leurs caractéristiques intrinsèques ces enzymes recombinantes diffèrent des enzymes retrouvées dans les microsomes de foie (séquence protéique tronquée, environnement membranaire distinct, couplage différent entre les cytochromes b5 et P450, absence de compétition entre les différents CYP450).

Les inhibiteurs biologiques, tels que les anticorps monoclonaux, dirigés contre une enzyme sont utilisés, après co-incubation du principe actif avec les microsomes de foie humain (versus un témoin sans anticorps) pour une estimation quantitative du métabolisme du principe actif. Cependant un nombre non négligeable d'anticorps utilisés montrent un manque de spécificité et de puissance d'inhibition. Enfin cette technique est lourde à mettre en place pour le phénotypage de voies métaboliques.

Les inhibiteurs chimiques des CYP450 permettent de déterminer la contribution de chaque cytochrome P450 directement par le pourcentage d'inhibition du métabolisme du principe actif si l'inhibition est totale et spécifique de l'enzyme étudiée. Les inhibiteurs peuvent se fixer de manière réversible ou non réversible à l'enzyme. Ils sont utilisés en co-incubation ou pré-incubation avec le principe actif et les microsomes de foie humain (versus une condition témoin sans inhibiteur), modèle représentatif de l'*in vivo* pour les voies oxydatives de métabolisme des principes actifs.
Le niveau d'inhibition liée aux inhibiteurs réversibles de type compétitifs (les plus fréquents) est dépendant des conditions d'incubations telles que le temps d'incubation et de la concentration en substrat. De plus, de nombreux inhibiteurs de CYP450 utilisés communément pour les études de phénotypage ne sont pas spécifiques d'un seul CYP450 (ex : kétoconazole, quercetine...). En conséquence, les inhibiteurs réversibles ne conviennent pas au phénotypage des cytochromes P450.

Afin de pallier aux inconvénients de ces méthodes, les inhibiteurs non réversibles ou irréversibles sont utilisés en vue d'obtenir une méthode quantitative robuste et représentative des conditions *in vivo.* L'inhibition est dite irréversible dans la mesure où l'enzyme ne retrouve jamais son activité, on parle d'inhibition « suicide ». Les inhibiteurs non réversibles sont oxydés par les cytochromes P450 en métabolites intermédiaires se liant irréversiblement aux enzymes. Ce processus est qualifié de MBI pour Mechanism-Based Inhibition car la molécule de départ n'est pas inhibitrice mais nécessite au moins un cycle catalytique de l'enzyme avant d'être activée en métabolite réactif se liant de manière covalente à ladite enzyme. Une inhibition MBI se caractérise par une inhibition irréversible du cytochrome P450 étudié et ne dépend pas de la concentration en substrat. Ces inhibiteurs suivent une cinétique de premier ordre avec les constantes suivantes : k_{inact} correspond à la vitesse maximale d'inactivation de l'enzyme et K_{I} correspond à la concentration en inhibiteur à la moitié de la vitesse maximale d'inactivation.
Plusieurs conditions doivent être réunies pour obtenir une inhibition totale d'un CYP450 en utilisant un inhibiteur irréversible :
1) il est nécessaire d'utiliser une concentration en inhibiteur non réversible suffisante (dépendant de son K_{I}) ;
2) dépendant de son k_{inact}, le temps de pré-incubation de l'inhibiteur avec les microsomes de foies doit être suffisant pour entraîner suffisamment de cycles catalytiques d'inactivation ;
3) le temps de pré-incubation ne doit pas entraîner de déplétion en inhibiteur suicide tel que sa concentration deviendrait trop faible pour inhiber totalement le P450.
Si l'inhibition du CYP450 est totale et spécifique (aucun autre P450 inhibé) il est donc possible de déterminer de manière quantitative l'implication de chaque cytochrome P450 dans le métabolisme d'un principe actif. Le schéma expérimental utilisé est alors le suivant :

| Sequence | | Microsomes de foie | 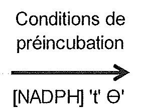 | Microsomes irréversiblement inhibés | 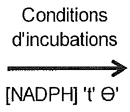 | f(t) =[PA] |
|---|---|---|---|---|---|---|
| Essai PA | Essai | + MBI | | + PA | | |
| | Témoin | - MBI | | + PA | | |
| Contrôle + Substrat spécifique | Essai | + MBI | | + Substrat | | |
| | Témoin | - MBI | | + Substrat | | |

En dépit des avantages cités ci-dessus, l'utilisation des MBI selon ce schéma expérimental présente un certains nombres de contraintes qui limitent son intérêt :
- d'une part, les conditions d'incubations du principe actif étudié sont dépendantes des conditions optimales de pré-incubation de l'inhibiteur non réversible (concentrations en protéines microsomales, pourcentage de solvant organique). En effet, afin de mesurer précisément le pourcentage d'inhibition du métabolisme du principe actif, ce dernier doit être incubé dans les conditions dites initiales (linéarité de la vitesse du métabolisme en fonction du temps et des concentrations en protéines, pourcentage de solvant ne peut excéder un certain niveau). Or, pour obtenir une inhibition maximale et spécifique dans ce schéma expérimental linéaire, l'inhibiteur doit lui-même être préalablement incubé dans des conditions *in vitro* de concentrations en protéines ou de solvant compatibles avec l'incubation du principe actif.
- d'autre part, le temps de pré-incubation altère l'activité enzymatique des CYP450 de microsomes qui présentent une demi-vie *in vitro* d'environ 70 à 90 minutes. On observe par exemple une perte d'activité enzymatique CYP2D6 jusqu'à 26% lors d'une pré-incubation de 30 minutes, jusqu'à 35% pour une pré-incubation de 40 minutes et 46% pour une pré-incubation de 60 minutes. En conséquence, la durée de la séquence continue de pré-incubation du MBI et d'incubation du candidat médicament imposée par le schéma expérimental est donc incompatible avec la demi-vie *in vitro* des CYP450 de microsomes.
- enfin, il est fréquent que les inhibiteurs irréversibles pour un CYP450 présentent également une inhibition réversible pour un ou plusieurs CYP450 (par exemple l'inhibiteur MBI du CYP1A2 est également inhibiteur compétitif du CYP2C19 et l'inhibiteur MBI du CYP2B6 est aussi inhibiteur compétitif des CYP2A6 et 3A4). Or le schéma expérimental linéaire ci-dessus ne permet pas d'éliminer de manière satisfaisante pour une étude de phénotypage *in vitro*, la part libre restante d'inhibiteur pouvant agir de manière compétitive sur d'autres CYP450. Une dilution du pré-incubat peut diminuer la concentration en inhibiteur libre, d'où moins d'inhibition réversible non spécifique, cependant elle va de facto diminuer la concentration en protéines microsomales de telle façon que le métabolisme du principe actif lors de l'incubation sera trop faible pour être détecté.
En conséquence, les difficultés pour rendre compatibles les conditions d'incubations de l'inhibiteur irréversible d'une part et du principe actif d'autre part, sont telles qu'il est rarement possible d'utiliser ce schéma expérimental linéaire.
De plus, lors de ce schéma expérimental linéaire, il est nécessaire d'ajouter deux séries d'incubations additionnelles (avec et sans inhibiteur irréversible) avec un substrat spécifique du CYP450 testé comme contrôle positif validant l'effet de l'inhibiteur. Ceci impose donc de quantifier non seulement le principe actif mais également l'ensemble des substrats spécifiques, et cela pour autant de CYP450 à tester, ce qui alourdi considérablement l'expérimentation.

Compte-tenu des inconvénients respectifs des méthodes de phénotypage des réactions enzymatiques impliquées dans le métabolisme d'un principe actif, une étude *in vitro* dudit phénotypage des réactions enzymatiques exige de surmonter les désavantages des différentes méthodes précédemment décrites.

La présente invention a donc pour but de proposer une stratégie alternative permettant de surmonter les problèmes inhérents à la mise en oeuvre d'une étude de phénotypage des réactions enzymatiques impliquées dans le métabolisme d'un principe actif par l'utilisation de microsomes isolés irréversiblement inhibés.

Cette invention a pour objet une méthode de préparation de microsomes isolés ayant un cytochrome P450 (CYP450) irréversiblement inhibé et comprenant les étapes suivantes :
- l'inhibition irréversible du cytochrome P450 ;
- la concentration des protéines microsomales.

Par inhibiteur non réversible, inhibiteur irréversible, inhibiteur MBI, inhibition irréversible ou inhibition suicide on entend un inhibiteur capable de se fixer de manière covalente à une enzyme ; l'enzyme ainsi inhibée ne peut retrouver son activité fonctionnelle initiale. De manière plus spécifique un inhibiteur MBI (Mechanism Based Inhibition) ne forme pas directement une liaison irréversible avec l'enzyme mais un de ses métabolites intermédiaires réactifs se lie de manière covalente à l'enzyme.

La concentration selon l'invention a pour but, d'une part de filtrer les protéines microsomales des autres produits présents dans la préparation notamment l'inhibiteur irréversible en excès restant libre ainsi que les solvants, d'autre part de concentrer les microsomes dilués par l'étape de pré-incubation.

La concentration des protéines microsomales selon l'invention peut être obtenue par filtration puis centrifugation. La fonction de filtration dans l'étape de concentration est obtenue à partir d'une membrane avec un seuil de coupure compris entre 10 000 Daltons et 40 000 Daltons, de préférence 30 000 Daltons. La fonction de concentration dans l'étape de concentration est réalisée par un système permettant la concentration, par exemple un système Centricon^{®} soumis à une centrifugation de 3000 g à 4000 g pendant 60 à 90 minutes, de préférence pendant 80 minutes. A la fin de cette étape, le Centricon^{®} est retourné et centrifugé de 800 à 1000 g pendant 2 à 10 minutes, de préférence pendant 5 minutes.
Afin d'améliorer la séparation et la concentration des protéines microsomales, une étape supplémentaire d'ultracentrifugation dans les conditions suivantes de 80 000 g à 150 000 g pendant environ 60 minutes peut être effectuée.
La concentration des protéines microsomales peut également être améliorée en répétant au moins deux fois la séquence : filtration puis concentration, décrite ci-dessus.

L'étape de concentration des protéines microsomales selon l'invention peut également être obtenue par ultracentrifugations successives. Au moins deux ultracentrifugations successives sont nécessaires dans les conditions suivantes de 80 000g à 150 000g pendant 60 à 90 minutes.

L'invention concerne une méthode de préparation de microsomes isolés irréversiblement inhibés dans laquelle une ou plusieurs étapes de lavage sont ajoutées. Cette méthode de préparation des microsomes isolés comprend des étapes de lavage placées avant et/ou après l'étape de concentration des protéines microsomales. Par exemple, la méthode selon l'invention comprend les étapes suivantes :
- l'inhibition irréversible d'un cytochrome P450 ;
- le lavage ;
- la concentration des protéines microsomales ;
- le lavage des protéines microsomales.

La méthode selon l'invention peut également comprendre les étapes suivantes :
- l'inhibition irréversible d'un cytochrome P450 ;
- la concentration des protéines microsomales ;
- le lavage des protéines microsomales ;
ou les étapes suivantes :
- l'inhibition irréversible d'un cytochrome P450 ;
- le lavage ;
- la concentration des protéines microsomales.

Par lavage on entend une étape rinçage et élimination des fractions non microsomales. L'étape de lavage peut consister également à reprendre le concentrat de microsomes dans du tampon Tris HCl à pH 7.4.

A l'issue de la méthode de préparation des microsomes isolés irréversiblement inhibés selon l'invention, les microsomes sont à une concentration de 10mg/ml à 30mg/ml, de manière plus spécifique de 17mg/ml à 25mg/ml et de préférence de 20mg/ml. Les protéines microsomales ont été concentrées par un facteur 5 à 15.

La méthode de préparation de microsomes isolés inactivés selon la présente invention comprend une étape finale de conservation des microsomes. Cette étape de conservation a pour but de faciliter l'utilisation *ex temporané* des microsomes inactivés selon l'invention. Cette utilisation des microsomes isolés peut avoir lieu de quelques jours à plusieurs mois après leur préparation. L'étape de conservation n'altère aucunement les propriétés structurelles et fonctionnelles des microsomes.
De manière préférée, l'étape de conservation est une étape de congélation. La congélation des microsomes isolés et irréversiblement inhibés est réalisée à -80°C.

Les microsomes utilisés au cours de la méthode de préparation sont des microsomes de foie humains, de rat, de souris, de porc ou de singe. De préférence, les microsomes sont issus de foies humains contenant l'ensemble des enzymes P450. Afin de tenir compte de la variabilité interindividuelle, les microsomes proviennent de plusieurs donneurs pour constituer un pool de microsomes.

Les cytochromes P450 sont une vaste famille d'isoenzymes constituée de 18 sous familles (CYP1, CYP2, CYP3, CYP4, CYP5, CYP7, CYP8, CYP11, CYP17, CYP19, CYP20, CYP21, CYP24, CYP26, CYP27, CYP39, CYP46, CYP51). La présente invention porte sur une méthode de préparation de microsomes isolés dont le cytochrome P450 irréversiblement inhibé est sélectionné parmi les familles de cytochrome CYP1, CYP2, CYP3 et CYP4.

Le cytochrome P450 irréversiblement inhibé est sélectionné parmi le CYP1A1, CYP1A2, CYP2A6, CYP2B6, CYP2C8, CYP2C9, CYP2C19, CYP2D6, CYP2E1, CYP2J2, CYP3A4, CYP3A5 et CYP4F2.

De préférence, le cytochrome P450 irréversiblement inhibé est sélectionné parmi le CYP1A2, CYP2A6, CYP2B6, CYP2C8, CYP2C9, CYP2C19, CYP2D6, CYP2E1 et CYP3A4.

Parmi les inhibiteurs non réversibles utilisés dans la méthode de préparation de microsomes selon l'invention on peut citer à titre indicatif et non limitatif :

| **CYP** | **Inhibiteurs MBI** | **Paramètres *in vitro (Littérature)*** | |
|---|---|---|---|
| | | ***Kₗ (µM)*** | ***k_{inact} (min-1)*** |
| 1A2 | Furafylline | 3-6 | 0.27-0.87 |
| 2A6 | 8 MethOxyPsoralene | 0.3-1.9 | 0.5-2.1 |
| | Menthofuran | 0.84-2.5 | 0.22-0.25 |
| | Sporalen | 0.6 | 0.3 |
| 2B6 | Thiotepa | 4.8-50 | 0.1-0.2 |
| | Phencyclidine | 10 | 0.01 |
| 2C8 | Gemfibrozil gluc. | 20 | 0.21 |
| 2C9 | Ac. Tienilique | 0.13-20 | 0.05-2 |
| | Suprofen | 3.7-26 | 0.07-0.09 |
| 2C19 | Fluoxetine | 0.2-54 | 0.06-0.1 |
| | Clopidogrel | 0.5-14.3 | 0.056-0.35 |
| | Ticlopidine | 1.65-87 | 0.19-0.032 |
| | Protopine | 7.1 | 0.24 |
| 2D6 | Paroxetine | 0.8-9.32 | 0.17 |
| | MDMA | 23 | 0.18 |
| | EMTPP | 5.5 | 0.09 |
| 2E1 | DiethylDithioCarbamate | 12.2 | 0.02 |
| | Phenethyl isothiocyanate | 9.98 | 0.33 |
| 3A4 | TroleAndOmycine | 2.4 | 0.032 |
| | Diltiazem | 8.7 | 0.005 |
| | Azamulin | 0.2 | 0.7 |

La présente invention porte également sur les microsomes isolés en tant que tels comprenant un cytochrome P450 irréversiblement inhibé.

Les microsomes isolés selon l'invention comprennent un cytochrome irréversiblement inhibé sélectionné parmi les familles de cytochromes CYP1, CYP2, CYP3 et CYP4.

L'invention concerne des microsomes isolés dont un cytochrome sélectionné dans la liste suivante est irréversiblement inhibé : CYP1A1, CYP1A2, CYP2A6, CYP2B6, CYP2C8, CYP2C9, CYP2C19, CYP2D6, CYP2E1, CYP2J2, CYP3A4, CYP3A5 et CYP4F2.

Plus particulièrement, l'invention concerne des microsomes isolés dont un cytochrome sélectionné dans la liste suivante est irréversiblement inhibé : CYP1A2, CYP2A6, CYP2B6, CYP2C8, CYP2C9, CYP2C19, CYP2D6, CYP2E1 et CYP3A4.

La présente invention concerne également les microsomes isolés irréversiblement inhibés sous une forme congelée pour une utilisation *ex temporané* après décongélation.

Les microsomes isolés sont obtenus selon les méthodes de préparation décrites dans la présente demande de brevet.

L'invention concerne l'utilisation des microsomes isolés irréversiblement inhibés dans le phénotypage des réactions enzymatiques impliquées dans le métabolisme d'un principe actif à évaluer.

L'invention porte également sur une méthode de phénotypage des réactions enzymatiques impliquées dans le métabolisme d'un principe actif. Cette méthode de phénotypage comprend les étapes suivantes :
- l'incubation de microsomes isolés et irréversiblement inhibés selon l'invention avec un principe actif à évaluer ;
- la mesure de la contribution du cytochrome P450 irréversiblement inhibé impliqué dans le métabolisme du principe actif.

L'incubation est réalisée en présence du principe actif avec d'une part les microsomes de foie isolés, irréversiblement inhibés selon l'invention et d'autre part les microsomes de foie isolés, non inhibés et préparés selon l'invention (témoin). L'incubation est suivie soit à un temps final d'incubation soit sous-forme d'une cinétique, i.e. plusieurs temps d'incubation.
A chaque temps d'incubation, le principe actif et/ou ses métabolites sont quantifiés.
L'activité métabolique (A) du principe actif est mesurée soit via la clairance intrinsèque métabolique (dans le cas d'une cinétique) soit via la vitesse de disparition de principe actif inchangé ou d'apparition de métabolites (si un seul temps final d'incubation a été prévu) dans les deux conditions, inhibée (activité Ai) et non inhibée (témoin avec l'activité A).
Le pourcentage d'inhibition de l'activité métabolique du principe actif se calcule de la manière suivante : pourcentage d'inhibition = (A-Ai)/A. Le pourcentage d'inhibition calculé correspond directement à la contribution du cytochrome P450 irréversiblement inhibé impliqué dans le métabolisme du principe actif.

Enfin l'invention concerne un kit de phénotypage comprenant :
- des microsomes isolés et irréversiblement inhibés selon l'invention ;
- des microsomes témoins.
On entend par microsomes témoins, des microsomes ayant subi la méthode de préparation selon l'invention en l'absence de l'inhibiteur irréversible.

La présente invention est illustrée sans pour autant être limitée par les figures et exemples suivants :

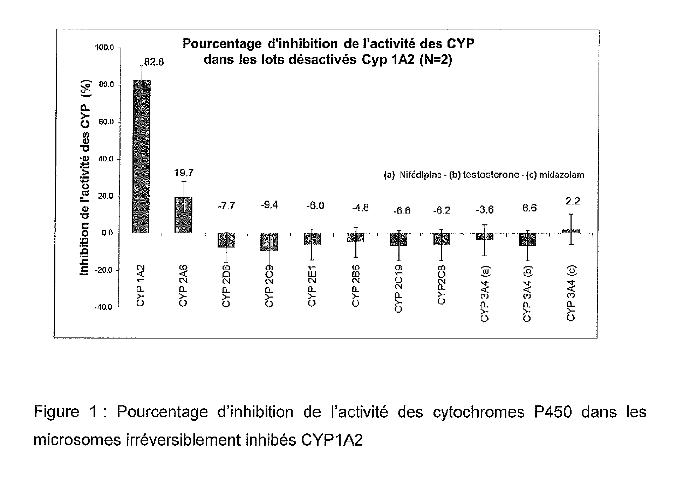

Figure 1 : Pourcentage d'inhibition de l'activité des cytochromes P450 dans les microsomes irréversiblement inhibés CYP1A2.
   Les activités spécifiques étudiées pour chaque CYP450 correspondent aux activités de phénacétine-O-déacéthylase (CYP1A2, incubation de phénacétine à 4.5 µM), coumarin-7-hydroxylase (CYP2A6, incubation de coumarine à 2 µM), bupropion-hydroxylase (2B6, incubation de bupropion à 50 µM), paclitaxel-6α-hydroxylase (2C8, incubation de paclitaxel à 4 µM), diclofenac-4'-hydroxylase (2C9, incubation de diclofenac à 4 µM), omeprazole-5-hydroxylase (2C19, incubation d'oméprazole à 5 µM), dextromethorphan-O-déméthylase (2D6, incubation de dextromethorphan à 5 µM), chlorzoxazone-6-hydroxylase (2E1, incubation de chlorzoxazone à 40 µM) et testostérone-6ß-hydroxylase, midazolam-1'-hydroxylase et nifédipine-réductase (3A4, incubation de testostérone à 30 µM, midazolam à 0.5 µM, nifédipine à 10 µM). Le pourcentage d'inhibition est obtenu par comparaison des activités P450 sur microsomes inhibés irréversiblement par la furafylline et sur les microsomes témoins.
Figure 2 : Impact de la furafylline à 5 µM et 10 µM sur les microsomes de foies humains et sur les microsomes recombinants pour le CYP1A2.
Figure 3 : Pourcentage d'inhibition de l'activité des cytochromes P450 dans les microsomes irréversiblement inhibés CYP3A4.
   Les activités spécifiques étudiées pour chaque CYP450 correspondent à celles décrites à la Figure 1. Le pourcentage d'inhibition est obtenu par comparaison des activités P450 sur microsomes inhibés irréversiblement par l'azamuline et sur les microsomes témoins.
Figure 4 : Métabolisme de la nifedipine et du midazolam par le CYP3A4 et le CYP3A5 (microsomes recombinants).
Figure 5 : Pourcentage d'inhibition de l'activité midazolam au Km et au Vmax dans les microsomes irréversiblement inhibés CYP3A4.
Figure 6 : Pourcentage d'inhibition de l'activité des cytochromes P450 dans les microsomes irréversiblement inhibé CYP2C8.
   Les activités spécifiques étudiées pour chaque CYP450 correspondent à celles décrites à la Figure 1 auxquelles s'ajoute l'activité de l'amodiaquine hydroxylase (2C8, incubation d'amodiaquine à 0.5 µM). Le pourcentage d'inhibition est obtenu par comparaison des activités P450 sur microsomes inhibés irréversiblement par le gemfibrozil glucuronide et sur les microsomes témoins.

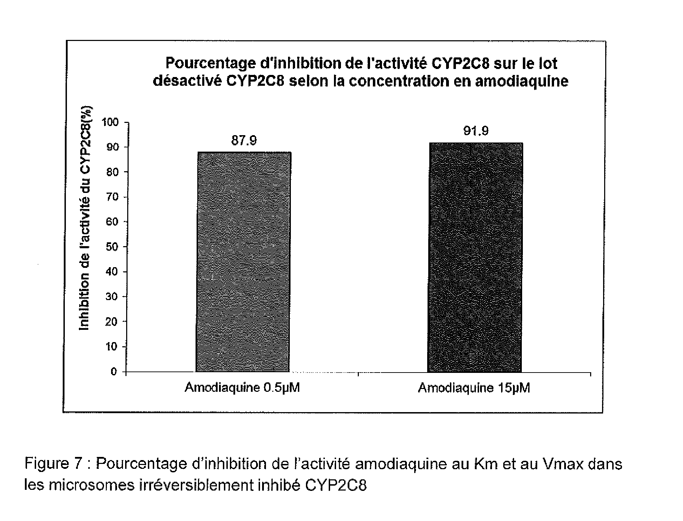

Figure 7 : Pourcentage d'inhibition de l'activité amodiaquine incubé aux concentrations correspondant au Km et au Vmax de la réaction 2C8 dépendante de l'amodiaquine hydroxylation, dans les microsomes irréversiblement inhibé CYP2C8.

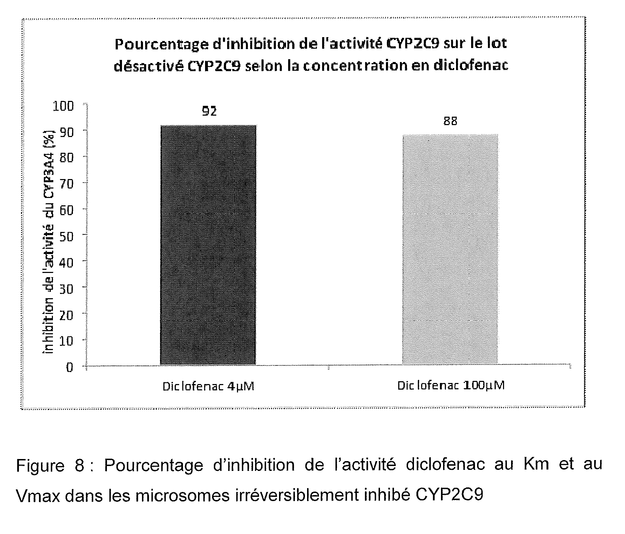

Figure 8 : Pourcentage d'inhibition de l'activité diclofenac incubé aux concentrations correspondant au Km et au Vmax de la réaction 2C9 dépendante de la diclofenac-4'-hydroxylation dans les microsomes irréversiblement inhibé CYP2C9.

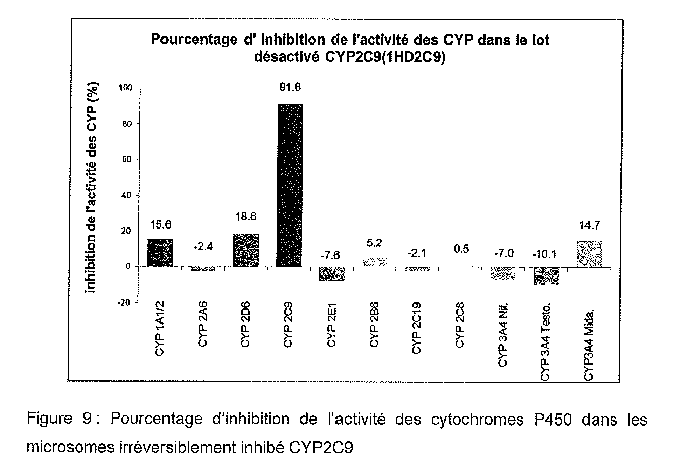

Figure 9 : Pourcentage d'inhibition de l'activité des cytochromes P450 dans les microsomes irréversiblement inhibé CYP2C9.
   Les activités spécifiques étudiées pour chaque CYP450 correspondent à celles décrites à la Figure 1. Le pourcentage d'inhibition est obtenu par comparaison des activités P450 sur microsomes inhibés irréversiblement par l'acide tienilique et sur les microsomes témoins.

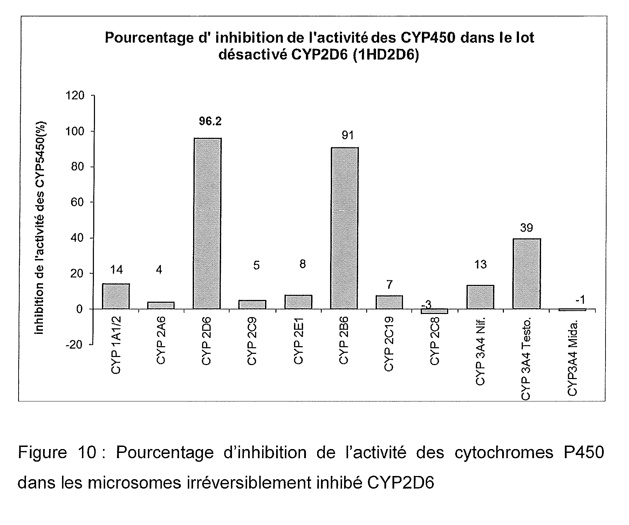

Figure 10 : Pourcentage d'inhibition de l'activité des cytochromes P450 dans les microsomes irréversiblement inhibé CYP2D6. Les activités spécifiques étudiées pour chaque CYP450 correspondent à celles décrites à la Figure 1. Le pourcentage d'inhibition est obtenu par comparaison des activités P450 sur microsomes inhibés irréversiblement par la paroxetine et sur les microsomes témoins.

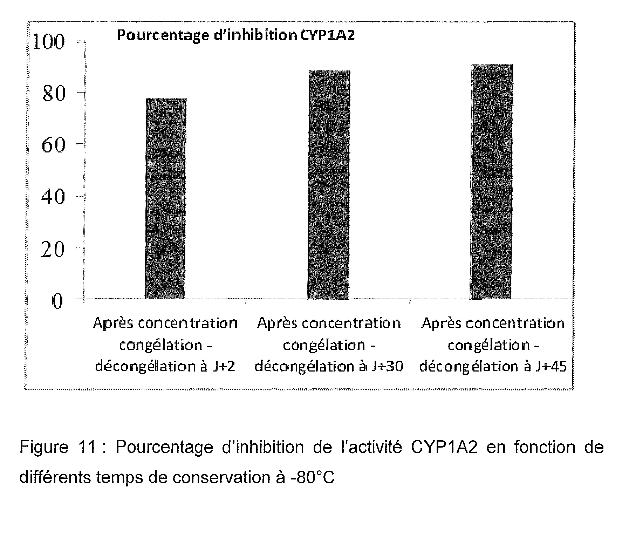

Figure 11 : Pourcentage d'inhibition de l'activité CYP1A2 en fonction de différents temps de conservation à -80°C

### Exemple 1 : préparation de microsomes irréversiblement inactivés

### • Matériel biologique

Les microsomes sont issus de foies humains contenant l'ensemble des enzymes P450. Ils proviennent d'un pool de microsomes puisqu'ils sont issus de plusieurs donneurs afin de tenir compte de la variabilité interindividuelle.

### • Incubation des microsomes

Pour chaque préparation de lot de microsomes irréversiblement inhibés, un lot témoin est préparé dans les mêmes conditions à la différence que l'inhibiteur irréversible est remplacé par un volume équivalent de solvant.

L'inhibiteur non réversible du cytochrome P450 étudié (ou le solvant, pour le lot témoin) est incubé avec les microsomes dans du tampon Tris/HCl à pH 7.4 et du MgCl₂ sous agitation à 37°C. La préparation est généralement préchauffée entre 5 et 10 minutes puis on ajoute du NADPH pour démarrer la réaction enzymatique. Au temps t, le milieu réactionnel est placé pendant quelques minutes dans la glace avant de passer à l'étape de concentration. Des complexes enzyme-inhibiteur liés par liaisons covalentes sont formés au cours de l'incubation des microsomes avec l'inhibiteur irréversible. Le cytochrome P450 étudié est inhibé de manière irréversible, totale et spécifique.

### • Filtration et concentration des microsomes irréversiblement désactivés

L'échantillon issu de l'incubation des microsomes avec l'inhibiteur non réversible du cytochrome P450 étudié est filtré. Cette étape de filtration des protéines peut être réalisée grâce à une membrane avec un seuil de coupure de 10 000 à 40 000 Daltons. Un système Centricon^{®} est utilisé pour effectuer cette étape de filtration.

Une ou plusieurs étapes de lavage sont parfois nécessaire pour faciliter l'élimination de l'inhibiteur irréversible restant libre. L'échantillon est centrifugé entre 3000 g à 4000 g pendant 80 minutes puis 800 à 1000 g pendant 5 minutes. L'échantillon peut subir une succession de centrifugations afin d'optimiser la concentration des protéines microsomales.

Le cas échéant, l'échantillon de microsomes concentré est ensuite ultracentrifugé afin d'améliorer encore la concentration des protéines. L'ultracentrifugation est réalisée dans une gamme de conditions comprises entre 80 000 g pendant 4 heures à 150 000g pendant 45 minutes, préférentiellement à 100 000 g pendant 1 heure.
Le concentrat de protéines est repris dans un tampon Tris/HCl à pH 7.4 puis aliquoté et congelé à -80°C.

### Exemple 2 : Conditions d'inhibition des principaux cytochromes P450 et validation des microsomes irréversiblement inhibés dans le phénotypage enzymatique des voies métaboliques

### • CYP1A2

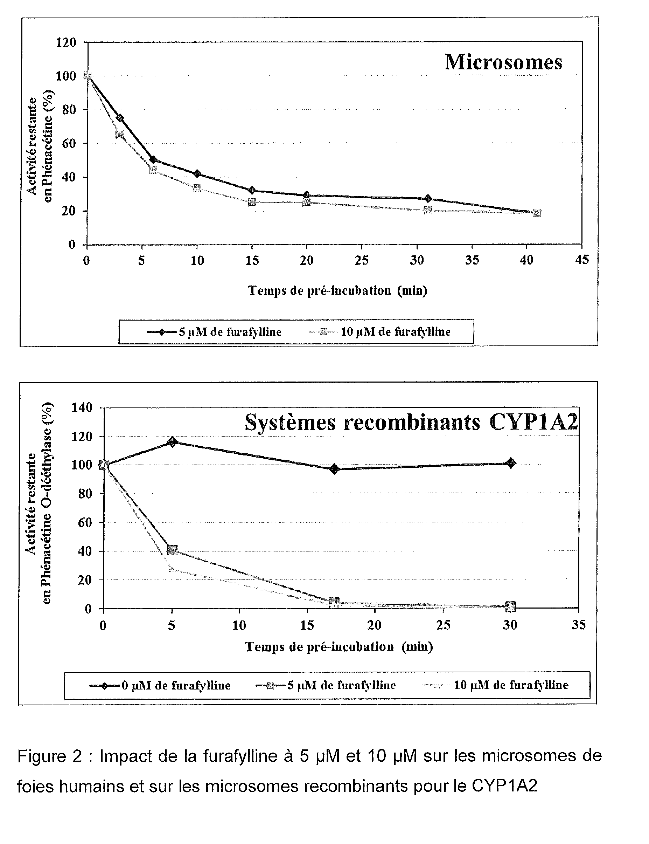

La furafylline est un des inhibiteurs MBI du cytochrome CYP1A2.
Les conditions expérimentales pour une inhibition MBI maximale par la furafylline sur le CYP1A2 sont les suivantes :
- protéines microsomales à 2 mg/ml ;
- furafylline à 10 µM ;
- temps d'incubation de 30 minutes.
Après incubation de la phénacétine, substrat spécifique du CYP1A2, à 4.5 µM (concentration inférieure ou égale à son Km) avec des microsomes préalablement inhibés selon les conditions ci-dessus, le pourcentage d'inhibition de l'activité phénacétine déacethylase (CYP1A1/CYP1A2 dépendante) est de 83% (Figure 1). Notons que les 17% de métabolisme restant sont dû à l'activité phénacétine déacethylase résiduelle liée au CYP1A1 et non à un défaut d'inhibition du CYP1A2. En effet il est connu que la phénacétine incubée dans des conditions non saturantes (< 5 µM) est métabolisée majoritairement par le CYP1A2 et partiellement par le CYP1A1.
La furafylline préincubée pendant 30 minutes de 5 µM à 10 µM avec des CYP1A2 recombinants humains entraîne 100% d'inhibition de l'activité purement CYP1A2 de la phénacétine prouvant ainsi sa puissance maximale d'inhibition aux conditions retenues (Figure 2).
Lorsque la phénacétine est incubée à une concentration très supérieure à son Km pour le CYP1A2 en présence des microsomes irréversiblement inhibés et du lot témoin préparés selon les conditions ci-dessus, le pourcentage d'inhibition de l'activité phénacétine déacéthylase (CYP1A1/CYP1A2 dépendante) est toujours d'environ 80%. Ce résultat prouve que l'inhibition du CYP1A2 par la furafylline n'est pas affectée par un excès de substrat et qu'aucune inhibition de type compétitive n'est détectable.
Les substrats spécifiques des autres CYP450 majoritaires (CYP2A6, 2B6, 2C8, 2C9, 2C19, 2D6, 2E1, et 3A4) ont été incubés en présence du lot de microsomes de foies humains irréversiblement inhibés par la furafylline et du lot témoin dans les conditions définies ci-dessus afin de démontrer la spécificité de la furafylline. On observe que l'activité des autres CYP450 majoritaires reste inchangée.
L'ensemble de ces résultats montrent que les microsomes isolés et irréversiblement et spécifiquement inhibés vis-à-vis du CYP1A2 selon l'invention peuvent valablement être utilisés pour mesurer la contribution du CYP1A2 dans le métabolisme d'un principe actif ou d'un candidat médicament.

### • CYP3A4

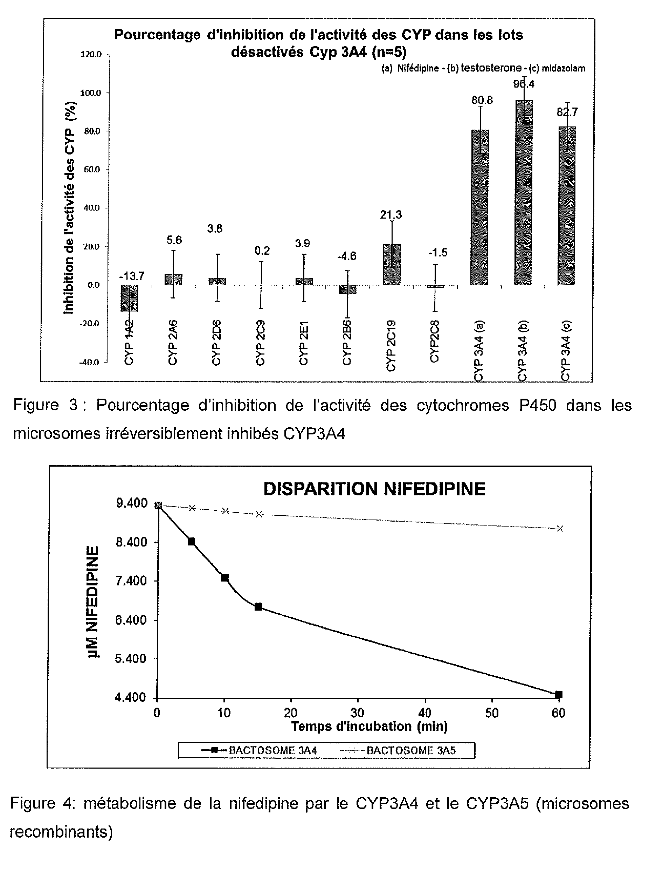

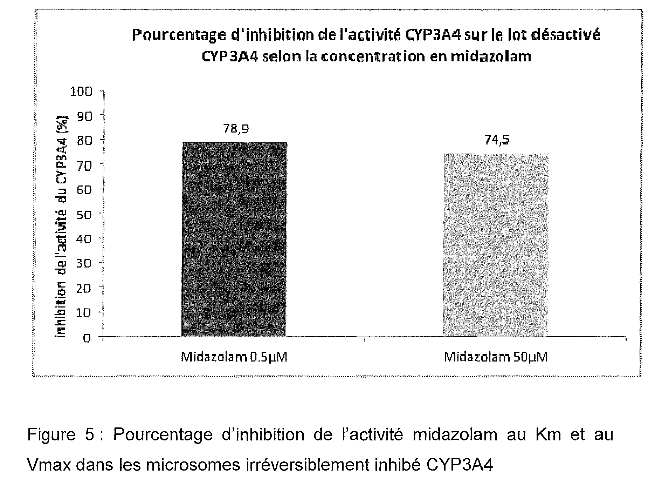

L'azamulin est un des inhibiteurs MBI du cytochrome CYP3A4.
Les conditions expérimentales pour une inhibition MBI maximale par l'azamulin sur le CYP3A4 sont les suivantes :
- protéines microsomales à 2 mg/ml ;
- azamulin à 5 µM ;
- temps d'incubation de 15 minutes.
Après incubation du midazolam à 0.5 µM, de la testostérone à 30 µM et de la nifédipine à 10 µM (concentrations inférieures ou égales au Km des substrats), substrats spécifiques du CYP3A4, en présence des microsomes irréversiblement inhibés et du lot témoin préparés selon les conditions ci-dessus détaillées, les pourcentages d'inhibition des activités midazolam-1'-hydroxylase, testosterone-6ß-hydroxylase, et nifédipine-réductase (CYP3A4/CYP3A5 dépendantes) sont respectivement de 81%, 96% et 83% (Figure 3). Notons que les 19%, 4%, 17% de métabolisme restant pour chacune de ces activités sont liés à l'activité CYP3A5 et non à un défaut d'inhibition du CYP3A4. En effet il est connu que les trois substrats spécifiques (et plus particulièrement nifedipine et midazolam) sont majoritairement métabolisés par le CYP3A4 mais également par le CYP3A5. La figure 4 montre notamment le métabolisme de la nifedipine par des microsomes recombinants (bactosomes) CYP3A4 et CYP3A5. L'azamulin pré-incubée pendant 15 minutes à 5 µM avec des CYP3A4 recombinants humains entraîne 92 % d'inhibition de l'activité purement CYP3A4 du midazolam prouvant sa puissance maximale d'inhibition aux conditions retenues. Lorsque les substrats spécifiques du CYP3A4 (exemple du midazolam) sont incubés à une concentration très supérieure à leur Km pour le CYP3A4 avec des microsomes préalablement préparés selon la présente invention, le pourcentage d'inhibition des activités CYP3A4 dépendantes reste inchangé (Figure 5). Ce résultat prouve que l'inhibition du CYP3A4 par l'azamulin n'est pas affectée par un excès de substrat et qu'aucune inhibition de type compétitive n'est détectable.
Les substrats spécifiques des autres CYP450 majoritaires (CYP1A2, CYP2A6, 2B6, 2C8, 2C9, 2C19, 2D6, et 2E1) ont été incubés en présence du lot de microsomes de foies humains irréversiblement inhibés par l'azamulin et du lot témoin dans les conditions définies ci-dessus afin de démontrer la spécificité de l'azamulin. La figure 3 montre que l'activité des autres CYP450 majoritaires reste inchangée entre le lot de microsomes humains irréversiblement inhibés et le lot témoin démontrant que l'azamulin est bien spécifique de l'activité CYP3A4.
L'ensemble de ces résultats montrent que les microsomes isolés et irréversiblement inhibés vis-à-vis du CYP3A4 selon l'invention peuvent valablement être utilisés pour mesurer la contribution du CYP3A4 dans le métabolisme d'un principe actif ou d'un candidat médicament.

### • CYP2C8

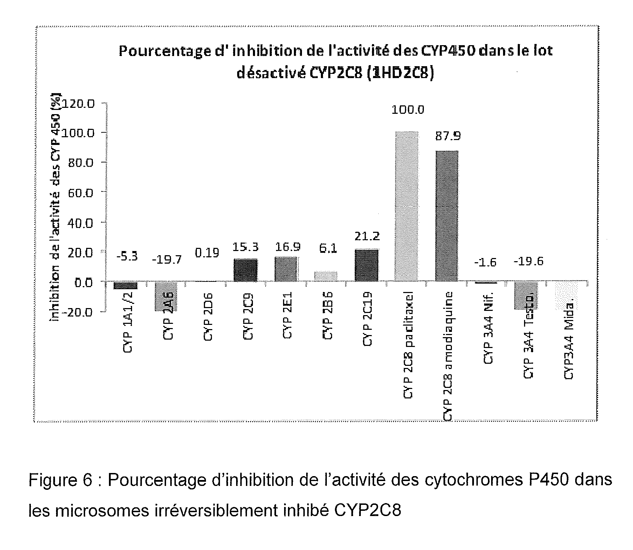

Le gemfibrozil glucuronide est un des inhibiteurs MBI du cytochrome CYP2C8. Les conditions expérimentales pour une inhibition MBI maximale par le gemfibrozil glucuronide sur le CYP2C8 sont les suivantes :
- protéines microsomales à 2 mg/ml ;
- gemfibrozil glucuronide à 30 µM ;
- temps d'incubation de 30 minutes.
Après incubation de l'amodiaquine à 0.5 µM ou du paclitaxel à 4 µM (concentrations inférieures ou égales au Km des deux substrats pour le CYP2C8), substrats spécifiques du CYP2C8, en présence des microsomes irréversiblement inhibés et du lot témoin préparés selon la présente invention, le pourcentage d'inhibition des activités amodiaquine (CYP2C8) et paclitaxel-hydroxylase (CYP2C8 dépendante) est respectivement de 88% et 100% (Figure 6).
Lorsque l'amodiaquine est incubée à une concentration très supérieure à son Km pour le CYP2C8 dans les mêmes conditions que celles décrites supra, le pourcentage d'inhibition de l'activité amodiaquine hydroxylase reste inchangée (Figure 7). Ce résultat prouve que l'inhibition du CYP2C8 par l'amodiaquine n'est pas affectée par un excès de substrat et qu'aucune inhibition de type compétitive n'est détectable.
Les substrats spécifiques des autres CYP450 majoritaires ont été incubés dans les conditions définies ci-dessus afin de démontrer la spécificité de la gemfibrozil glucuronide. La figure 6 montre que l'activité des autres CYP450 majoritaires reste inchangée à l'exception d'une légère inhibition du CYP2C19 entre le lot de microsomes humains irréversiblement inhibé et le lot témoin démontrant que le gemfibrozil glucuronide est bien spécifique de l'activité CYP2C8.
L'ensemble de ces résultats montrent que les microsomes isolés et irréversiblement inhibés vis-à-vis de l'activité CYP2C8 selon l'invention peuvent valablement être utilisés pour mesurer la contribution du CYP2C8 dans le métabolisme d'un principe actif ou d'un candidat médicament.

### • CYP2C9

L'acide tienilique est un des inhibiteurs MBI du cytochrome CYP2C9.
Les conditions expérimentales pour une inhibition MBI maximale par l'acide tienilique sur le CYP2C9 sont les suivantes :
- protéines microsomales à 2 mg/ml ;
- acide tienilique à 10 µM ;
- temps d'incubation de 20 minutes.
Après incubation du diclofenac, substrat spécifique du CYP2C9, à 4 µM (concentration inférieure ou égale au Km du substrat pour le CYP2C9) et 100 µM (concentration très supérieure à son Km pour le CYP2C9) en présence des microsomes irréversiblement inhibé et du lot témoin préparés selon la présente invention, l'inhibition de l'activité diclofenac hydroxylase (CYP2C9 dépendante) est quasi-totale soit respectivement 92% et 88% d'inhibition (Figure 8). Ce résultat prouve que non seulement l'inhibition du CYP2C9 est totale mais aussi qu'elle n'est pas affectée par un excès de substrat et qu'aucune inhibition de type compétitive n'est détectable.
Les substrats spécifiques des autres CYP450 majoritaires ont été incubés dans les conditions définies ci-dessus afin de démontrer la spécificité de l'acide tienilique. La figure 9 montre que l'activité des autres CYP450 majoritaires reste inchangée entre le lot de microsomes irréversiblement inhibé et le lot témoin démontrant que l'acide tienilique est bien spécifique de l'activité CYP2C9.
L'ensemble de ces résultats montrent que les microsomes isolés et irréversiblement inhibés vis-à-vis de l'activité CYP2C9 selon l'invention peuvent valablement être utilisés pour mesurer la contribution du CYP2C9 dans le métabolisme d'un principe actif ou d'un candidat médicament.

### • CYP2D6

La paroxetine est un des inhibiteurs MBI du cytochrome CYP2D6.
Les conditions expérimentales pour une inhibition MBI maximale par la paroxetine sur le CYP2D6 sont les suivantes :
- protéines microsomales à 2mg/ml ;
- paroxetine à 50 µM ;
- temps d'incubation de 30 minutes.
Après incubation du dextromethorphan, substrat spécifique du CYP2D6, à 5 µM (concentration inférieure ou égale au Km du substrat) et 50 µM (concentration très supérieure à son Km pour le CYP2D6) en présence des microsomes irréversiblement inhibé et du lot témoin préparés selon la présente invention, l'inhibition de l'activité dextromethorphan-O-demethylase (CYP2D6 dépendante) est quasi-totale soit 96% d'inhibition (Figure 10). Ce résultat prouve que l'inhibition du CYP2D6 est totale, qu'elle n'est pas affectée par un excès de substrat et qu'aucune inhibition de type compétitive n'est détectable.
Les substrats spécifiques des autres CYP450 majoritaires ont été incubés dans les conditions définies ci-dessus afin de démontrer la spécificité de la paroxétine. La figure 10 montre que parmi toutes les autres activités CYP450, seule l'activité bupropion hydroxylase dépendante du CYP2B6 est inhibée à 91% en supplément du CYP2D6 démontrant que la paroxétine n'est pas totalement spécifique de l'activité CYP2D6.
L'ensemble de ces résultats montrent que des microsomes préparés selon l'invention permettent d'inhiber totalement et quasi spécifiquement l'activité CYP2D6. Ils peuvent donc être utilisés pour mesurer la contribution du CYP2D6/CYP2B6 dans le métabolisme d'un nouveau candidat médicament.

### Exemple 3 : Stabilité de l'inhibition des cytochromes P450 dans les microsomes isolés, irréversiblement inhibés et conservés par congélation

Des microsomes isolés de foies humains irréversiblement inhibés en CYP1A2 ayant été concentrés et conservés à -80°C conformément à l'invention sont incubés pendant 15 minutes avec de la phénacétine (4.5µM) substrat spécifique de CYP1A2 à 1mg/mL. Les pourcentages d'inhibition ont été mesurés pour des microsomes obtenus selon l'invention et conservés à -80°C pendant 48 heures, un mois et un mois et demi (Figure 11). On observe que les étapes de concentration, congélation-décongélation n'altèrent pas l'inhibition MBI du CYP1A2 par la furafylline. Les étapes de congélation et décongélation n'ont pas d'influence sur la stabilité de l'inhibition irréversible des cytochromes P450.

## Revendications

1. Méthode de préparation de microsomes isolés comprenant un cytochrome P450 (CYP450) irréversiblement inhibé **caractérisé en ce qu'**elle comprend les étapes suivantes :
a) l'inhibition irréversible d'un cytochrome P450 ;
b) la concentration des protéines microsomales.

2. Méthode selon la revendication 1, **caractérisée en ce qu'**elle comprend une à plusieurs étapes de lavage.

3. Méthode selon la revendication 2, **caractérisée en ce que** la ou les étapes de lavage sont placées avant et/ou après l'étape de concentration des protéines microsomales.

4. Méthode selon la revendication 1, **caractérisée en ce que** la concentration des microsomes est obtenue par filtration/centrifugation ou ultracentrifugations.

5. Méthode selon la revendication 1, **caractérisée en ce que** les microsomes sont concentrés à une concentration de 10mg/ml à 30mg/ml.

6. Méthode selon la revendication 1, **caractérisée en ce qu'**elle comprend une étape finale de conservation.

7. Méthode selon la revendication 6, **caractérisée en ce que** l'étape de conservation est la congélation.

8. Méthode selon la revendication 1, **caractérisée en ce que** les microsomes sont des microsomes de foie humains.

9. Méthode selon la revendication 1, **caractérisée en ce que** le cytochrome P450 irréversiblement inhibé est sélectionné dans les familles CYP1A, CYP2A et CYP3A.

10. Méthode selon la revendication 9, **caractérisée en ce que** le cytochrome P450 est sélectionné parmi la liste des cytochromes suivants : CYP1A2, CYP2A6, CYP2B6, CYP2C8, CYP2C9, CYP2C19, CYP2D6, CYP2E1, CYP3A4.

11. Microsome isolé **caractérisé en ce que** le cytochrome P450 est irréversiblement inhibé.

12. Microsome isolé selon la revendication 11, **caractérisé en ce que** le cytochrome P450 irréversiblement inhibé est sélectionné dans les familles CYP1A, CYP2A et CYP3A.

13. Microsome isolé selon la revendication 12, **caractérisé en ce que** le cytochrome P450 est sélectionné parmi la liste des cytochromes suivants : CYP1A2, CYP2A6, CYP2B6, CYP2C8, CYP2C9, CYP2C19, CYP2D6, CYP2E1, CYP3A4.

14. Microsome selon l'une des revendications 11 à 13, **caractérisé en ce qu'**il est conservé par congélation.

15. Microsome isolé **caractérisé en ce qu'**il est obtenu selon la méthode de préparation selon l'une quelconque des revendications 1 à 10 de l'invention.

16. Méthode de phénotypage des réactions enzymatiques impliquées dans le métabolisme d'un principe actif, **caractérisée en ce qu'**elle comprend les étapes suivantes :
- l'incubation de microsomes isolés selon les revendications 11 à 14 avec un principe actif ;
- la mesure de la contribution du cytochrome P450 irréversiblement inhibé impliqué dans le métabolisme du principe actif.

17. Kit de phénotypage caractérisé en qu'il comprend :
- des microsomes isolés selon les revendications 11 à 14 ;
- des microsomes témoins.
